# EUROPEAN PATENT SPECIFICATION

(11) **EP 3 107 591 B1**
(45) Date of publication and mention of the grant of the patent: **18.04.2018**
(21) Application number: 15705823.1
(22) Date of filing: 20.02.2015
(51) Int. Cl.: A61L 27/36, A61L 27/54, C12N 5/077

(54) **FGF-18 IN GRAFT TRANSPLANTATION AND TISSUE ENGINEERING PROCEDURES**
FGF-18 BEI IMPLANTATTRANSPLANTATIONS- UND GEWEBEZÜCHTUNGSVERFAHREN
FGF-18 DANS DES PROCÉDURES DE TRANSPLANTATION ET LE GÉNIE TISSULAIRE

(30) Priority: 20.02.2014 EP 14000599
(43) Date of publication of application: 28.12.2016
(73) Proprietor: Merck Patent GmbH, 64293 Darmstadt (DE)
(72) Inventor: LADEL, Christoph H., 64291 Darmstadt (DE); GUEHRING, Hans, 65343 Eltville (DE); GIGOUT, Anne, 64289 Darmstadt (DE)
(74) Representative: Merck Serono S.A. Intellectual Property
(86) International application number: PCT/EP2015/053639
(87) International publication number: WO 2015/124735

(56) References cited:
- WO-A2-2004/032849
- WO-A2-2008/023063

### SEQUENCE LISTING

<110> Merck Patent GmbH
<120> FGF-18 in graft transplantation and tissue engineering procedures
<130> P14/015
<150> EP14000599.2
   <151> 20.02.2014
<160> 2
<170> PatentIn version 3.5
<210> 1
   <211> 207
   <212> PRT
   <213> Homo sapiens
<220>
   <223> human FGF-18
<400> 1
   Met Tyr Ser Ala Pro Ser Ala Cys Thr Cys Leu Cys Leu His Phe Leu
   1               5                   10                  15
   Leu Leu Cys Phe Gln Val Gln Val Leu Val Ala Glu Glu Asn Val Asp
               20                  25                  30
   Phe Arg Ile His Val Glu Asn Gln Thr Arg Ala Arg Asp Asp Val Ser
           35                  40                  45
   Arg Lys Gln Leu Arg Leu Tyr Gln Leu Tyr Ser Arg Thr Ser Gly Lys
       50                  55                  60
   His Ile Gln Val Leu Gly Arg Arg Ile Ser Ala Arg Gly Glu Asp Gly
   65                  70                  75                  80
   Asp Lys Tyr Ala Gln Leu Leu Val Glu Thr Asp Thr Phe Gly Ser Gln
                   85                  90                  95
   Val Arg Ile Lys Gly Lys Glu Thr Glu Phe Tyr Leu Cys Met Asn Arg
               100                 105                 110
   Lys Gly Lys Leu Val Gly Lys Pro Asp Gly Thr Ser Lys Glu Cys Val
           115                 120                 125
   Phe Ile Glu Lys Val Leu Glu Asn Asn Tyr Thr Ala Leu Met Ser Ala
       130                 135                 140
   Lys Tyr Ser Gly Trp Tyr Val Gly Phe Thr Lys Lys Gly Arg Pro Arg
   145                 150                 155                 160
   Lys Gly Pro Lys Thr Arg Glu Asn Gln Gln Asp Val His Phe Met Lys
                   165                 170                 175
   Arg Tyr Pro Lys Gly Gln Pro Glu Leu Gln Lys Pro Phe Lys Tyr Thr
               180                 185                 190
   Thr Val Thr Lys Arg Ser Arg Arg Ile Arg Pro Thr His Pro Ala
           195                 200                 205
<210> 2
   <211> 170
   <212> PRT
   <213> Artificial
<220>
   <223> Recombinant truncated FGF-18(trFGF-18)
<400> 2
   Met Glu Glu Asn Val Asp Phe Arg Ile His Val Glu Asn Gln Thr Arg
   1               5                   10                  15
   Ala Arg Asp Asp Val Ser Arg Lys Gln Leu Arg Leu Tyr Gln Leu Tyr
               20                  25                  30
   Ser Arg Thr Ser Gly Lys His Ile Gln Val Leu Gly Arg Arg Ile Ser
           35                  40                  45
   Ala Arg Gly Glu Asp Gly Asp Lys Tyr Ala Gln Leu Leu Val Glu Thr
       50                  55                  60
   Asp Thr Phe Gly Ser Gln Val Arg Ile Lys Gly Lys Glu Thr Glu Phe
   65                  70                  75                  80
   Tyr Leu Cys Met Asn Arg Lys Gly Lys Leu Val Gly Lys Pro Asp Gly
                   85                  90                  95
   Thr Ser Lys Glu Cys Val Phe Ile Glu Lys Val Leu Glu Asn Asn Tyr
               100                 105                 110
   Thr Ala Leu Met Ser Ala Lys Tyr Ser Gly Trp Tyr Val Gly Phe Thr
           115                 120                 125
   Lys Lys Gly Arg Pro Arg Lys Gly Pro Lys Thr Arg Glu Asn Gln Gln
       130                 135                 140
   Asp Val His Phe Met Lys Arg Tyr Pro Lys Gly Gln Pro Glu Leu Gln
   145                 150                 155                 160
   Lys Pro Phe Lys Tyr Thr Thr Val Thr Lys
                   165                 170

## Description

### Field of Invention

The present invention relates to regenerative medicine, in particular for the treatment of cartilage disorders, such as osteoarthritis, cartilage injury and osteochondral defects. More particularly, it relates to processes related to tissue engineering and graft procedures, such as osteochondral or cartilage transplantation or autologous chondrocyte implantation (ACI).

### Background of the invention

Cartilage disorders broadly refer to diseases characterized by degeneration of metabolic abnormalities in the connective tissues which are manifested by pain, stiffness and limitation of motion of the affected body parts. These disorders can be due to a pathology, e.g. osteoarthritis (OA), or can be the result of trauma or injury. Osteochondral defects (OCD), i.e. defect of the cartilage covering the end of a bone in a joint, are more often due to a trauma or injury, but can also be due to a pathology. OCD may lead to OA. Mature cartilage has limited ability to repair itself, notably because mature chondrocytes have little potential for proliferation and due to the absence of blood vessels. Replacement of damaged cartilage, in particular articular cartilage, caused either by injury or disease is a major challenge for physicians, and available surgical treatment procedures are considered unpredictable and effective for only a limited time. Therefore, the majority of younger patients either do not seek treatment or are counseled to postpone treatment for as long as possible. When treatment is required, the standard procedure is age dependend and varies between total joint replacement, transplantation of pieces of cartilage or marrow stimulating technique (such as microfracture). Microfracture is a cheap and common procedure that involves penetration of the subchondral bone to stimulate cartilage deposition by bone marrow derived stem cells. However, it has been shown that this technique does not repair sufficiently the chondral defect and the new cartilage formed is mainly fibrocartilage, resulting in inadequate or altered function. Indeed, fibrocartilage does not have the same durability and may not adhere correctly to the surrounding hyaline cartilage. For this reason, the newly synthesized fibrocartilage may breakdown more easily (expected time frame: 5-10 years).

For patients with osteoarthritis, non-surgical treatment consists notably of physical therapy, lifestyle modification (e.g. reducing activity), supportive devices, oral and injection drugs (e.g. non-steroidal anti-inflammatory drugs), and medical management (although there is not yet commercially available treatment that restores the cartilage damages (see Lotz, 2010)). Once these treatments fail, surgery, such as joint replacement (in part or totally), is the main option for the patients. Such an option can provide a reduction in symptoms but most often results in decreased joint function. Tibial or femoral osteotomies (cutting the bone to rebalance joint wear) may reduce symptoms, help to maintain an active lifestyle, and delay the need for total joint replacement. Total joint replacement can provide relief for the symptom of advanced osteoarthritis, but generally requires a change in the patient's lifestyle and/or activity level.

Current cartilage restorative procedures include total joint replacement, marrow stimulation (e.g. microfractures), osteochondral allografts or autografts, and cultured cartilage implantation (such as autologous chondrocyte implantation (ACI)). These procedures provide treatment options in particular for patients with a symptomatic chondral injury.

Osteochondral allograft or autograft transplantations are common procedures for the treatment of focal articular defects. Multiple factors likely influence the effectiveness of this procedure, including the source of donor cartilage, health of cartilage surrounding the defect site, and quality of integration. Unfortunately, in many cases, osteochondral transplantation procedures result in poor integration. Generally, for tissue engineering approach, cells are grown in a three-dimensional (3D) matrix, where each elements of said matrix play a key role in tissue regeneration. The main type of stem cells used for cartilage formation is human MSC (hMSC) (Zhang et al., 2013). However the type of MSC, scaffold, and other factors are important in tissue engineering. In addition, ensuring regeneration of a homogenous hyaline cartilage-like structure is important for high qulity integration into the defect. Establishing and maintaining said phenotype during articular cartilage tissue engineering is complex and may be optimized by using factors inhibiting the hypertrophy (Tang et al., 2012). For instance, although the addition of TGF-beta1 improved aggrecan, collagen type II and Sox-9 gene expression of hMSCs, but the newly synthesized cartilage mainly consist of fibrous, short-lasting tissue rather than hyaline tissue (Zhang et al., 2013).

Another type of tissue-engineering procedure is the cultured cartilage implantation procedure, such as autologous chondrocyte implantation (ACI), for which cartilage is taken from a low-weight bearing area of the articular surface of the patient to be treated; chondrocytes are then isolated and cultured *in vitro,* either in monolayer cultures or in 3D cultures; after a certain time in culture, the resulting chondrocytes or 3D constructs are implanted into the defect in order to fill in the defect. Unfortunately, the expansion of chondrocytes, notably in monolayer cultures, is known to induce fibroblast-like chondrocytes (Magill et al., 2011).

Fibroblast Growth factor 18 (FGF-18) is a proliferative agent for chondrocytes and osteoblasts (Ellsworth et al., 2002; Shimoaka et al., 2002). It has been proposed for the treatment of cartilage disorder such as osteoarthritis and cartilage injury either alone (WO2008023063) or in combination with hyaluronic acid (WO2004032849). Freeze-dried formulations containing FGF-18 have shown promising results in the treatment of OA or Cl, when injected intra-articularly.

Although cartilage restorative procedures such as osteochondral grafts, and cultured cartilage implantation (e.g. ACI) are promising, integration rate or quality of the cartilage produced have to be improved. There is therefore a need of a method for improved procedure, allowing good integration and good quality of the cartilage produced (i.e. mainly hyaline cartilage). Indeed, generation of said hyaline cartilage is valuable both as a therapeutic and as component for biological matrices (Getgood et al., 2010).

### Summary of the invention

It is an object of the present invention to provide a process for producing a transplantable cartilage material for tissue engineering or osteochondral/cartilage graft, wherein said process comprises or consists of the steps of: culturing chondrogenic cells, in 3D culture, or culturing osteochondral/cartilage explant(s), in a culture medium comprising an FGF-18 compound for a time sufficient to allow the formation of a transplantable osteochondral/cartilage material, wherein the FGF-18 compound is added intermittently in the culture medium, for about one, two or three days per week, said about one-day, two-days or three-days addition being repeated each week for at least 2 weeks of culture, at least 3 weeks of culture or at least 4 weeks of culture.

In an alternative, it is an object of the present invention to provide a process for producing a transplantable cartilage material for tissue engineering or osteochondral/cartilage graft, wherein said process comprises or consists of the steps of: culturing chondrogenic cells, in 3D culture, or culturing osteochondral/cartilage explant(s), in a culture medium comprising an FGF-18 compound for a time sufficient to allow the formation of a transplantable osteochondral/cartilage material, wherein the FGF-18 compound is added intermittently in the culture medium, for about one, two or three days per months, said about one-day, two-days or three-days addition being repeated each month for at least 2 months of culture, at least 3 months of culture or at least 4 months of culture.

Optionnaly, the FGF-18 compound can additionally be injected at the site of transplantation of the resulting osteochondral/cartilage material, either before, at the time of or after transplantation.

In the context of the present invention as a whole, the chondrogenic cells or the osteochondral/cartilage explant(s) are preferably harvested or isolated from a mammal before expansion or culture step.

In one embodiment, for chondrocytes or chondrogenic cell 3D culture or for osteochondral/cartilage explants(s), the FGF-18 compound is added intermittently in the culture medium, for about one day, 2 or 3 days per week, said one-day, 2- or 3-days addition being repeated each week for at least 2 weeks of culture, at least 3 weeks of culture or at least 4 weeks of culture. Preferably, said FGF-18 compound is added intermittently in the culture medium, for one, two or three days per week, said one-day, 2- or 3-days addition being repeated each week for 2 weeks of culture, 3 weeks of culture or 4 weeks of cultures. In an alternative embodiment, the FGF-18 compound is added intermittently in the culture medium, for about one, 2 or 3 days per month, said one-day, 2- or 3-days addition being repeated each month for at least 2 months of culture, at least 3 months of culture or at least 4 months of culture.

According to any one of the embodiments of the present invention, the cartilage disorder is preferably osteoarthritis, a cartilage injury or an osteochondral defect.

In the context of the present invention as a whole, the FGF-18 compound is preferably selected from the group consisting of: a) a polypeptide comprising or consisting of the human FGF-18 mature form comprising residues 28-207 of SEQ ID NO:1, b) a polypeptide comprising or consisting of the residues 28-196 of SEQ ID NO:1, or c) a polypeptide comprising or consisting of SEQ ID NO:2.

Further, in the context of the present invention as a whole, the explant is preferably a cartilage explants and the chondrogenic cells are preferably chondrocytes or mesenchymal stem cells derived from mature tissues. Depending on the need, the chondrogenic cells or the osteochondral/cartilage explants are harvest from the mammal to be treated or from a different mammal, preferably from the same species as the mammal to be treated. The mammal to be treated is preferably a human, but alternatively, and without any limitation, can also be a horse, a camel, a sheep, a dog or smaller mammals such as cats, rabbits, rats or mice.

### Definitions

- The term "FGF-18 compound" or "FGF-18", as used herein, is intended to be a protein maintaining at least one biological activity of the human FGF-18 protein. FGF-18 may be native, in its mature form, a recombinant form or a truncated form thereof. Biological activities of the human FGF-18 protein include notably the increase in chondrocyte or osteoblast proliferation (see WO98/16644) or in cartilage formation (see WO2008/023063). Native, or wild-type, human FGF-18 is a protein expressed by chondrocytes of articular cartilage. Human FGF-18 was first designated zFGF-5 and is fully described in WO98/16644. SEQ ID NO:1 corresponds to the amino acid sequence of the native human FGF-18, with a signal peptide consisting of amino acid residues 1 (Met) to 27(Ala). The mature form of human FGF-18 corresponds to the amino acid sequence from residue 28(Glu) to residue 207(Ala) of SEQ ID NO: 1 (180 amino acids).
   FGF-18, in the present invention, may be produced by recombinant method, such as taught by the application WO2006/063362. Depending on the expression systems and conditions, FGF-18 in the present invention is expressed in a recombinant host cell with a starting Methionine (Met) residue or with a signal sequence for secretion. When expressed in prokaryotic host, such as in E. coli, FGF-18 contains an additional Met residue in N-terminal of its sequence. For instance, the amino acid sequence of human FGF-18, when expressed in E.coli, starts with a Met residue in N-term (position 1) followed by residues 28 (Glu) to residue 207 (Ala) of SEQ ID NO: 1.
- The term "truncated form"of FGF18, as used herein, refers to a protein which comprises or consists of residues 28(Glu) to 196(Lys) of SEQ ID NO: 1. Preferably, the truncated form of FGF-18 protein is the polypeptide designated "trFGF-18" (170 amino acids; also known as rhFGF18 or sprifermin), which starts with a Met residue (in N-terminal) followed by amino acid residues 28 (Glu) -196 (Lys) of the wild-type human FGF-18. The amino acid sequence of trFGF-18 is shown in SEQ ID NO:2 (amino acid residues 2 to 170 of SEQ ID NO:2 correspond to amino acid residues 28 to 196 of SEQ ID NO:1). trFGF-18 is a recombinant truncated form of human FGF-18, produced in E.coli (see WO2006/063362). trFGF-18 has been shown to display similar activities as the mature human FGF-18, e.g. it increases chondrocyte proliferation and cartilage deposition leading to repair and reconstruction for a variety of cartilaginous tissues (see WO2008/023063).
- The term "cartilage disorder", as used herein, encompasses disorders resulting from damages due to injury, such as traumatic injury, chondropathy or arthritis. Such disorders result in a defect, more preferably in a cartilage defect. Examples of cartilage disorders that may be treated by the administration of the FGF-18 formulation described herein include but are not restricted to arthritis, such as osteoarthritis, cartilage injury and osteochondral defects. Degenerative diseases/disorders of the cartilage or of the joint, such as chondrocalcinosis, polychondritis, relapsing polychondritis, ankylosing spondylitis or costochondritis are also encompassed by this wording. The International Cartilage Repair Society has proposed an arthroscopic grading system to assess the severity of the cartilage defect: grade 0: (normal) healthy cartilage, grade 1: the cartilage has a soft spot or blisters, grade 2: minor tears visible in the cartilage, grade 3: lesions have deep crevices (more than 50% of cartilage layer) and grade 4: the cartilage tear exposes the underlying (subchronal) bone. (see the publication from ICRS: http://www.cartilage.org/_files/contentmanagement/ICRS_evaluation.pdf, page 13).

- The term "arthritis" as used herein encompasses disorders such as osteoarthritis, rheumatoid arthritis, juvenile rheumatoid arthritis, infectious arthritis, psoriatic arthritis, Still's disease (onset of juvenile rheumatoid arthritis) or osteochondritis dissecan. It preferably includes diseases or disorders in which ones the cartilage is damaged or detached from the underlying bone,
- The term "Osteoarthritis" is used to intend the most common form of arthritis. The term "osteoarthritis" is considered as a cartilage disorder which encompasses both primary osteoarthritis and secondary osteoarthritis (see for instance The Merck Manual, 17th edition, page 449). Osteoarthritis may be caused by the breakdown of cartilage. Bits of cartilage may break off and cause pain and swelling in the joint between bones. Over time, the cartilage may wear away entirely, and the bones will rub together. Osteoarthritis can affect any joint but usually concerns hands, shoulders and weight-bearing joints such as hips, knees, feet, and spine. In a preferred example, the osteoarthritis may be knee osteoarthritis or hip osteoarthritis. This wording encompasses notably the forms of osteoarthritis which are classified as stage 1 to stage 4 or grade 1 to grade 6 according to the OARSI classification system. The skilled person is fully aware of osteoarthritis classifications that are used in the art, in particular said OARSI assessment system (also named OOCHAS; see for instance Custers et al., 2007). Osteoarthritis is one of the preferred cartilage disorders that can be treated by administering the FGF-18 compounds according to the present invention.
- The term "cartilage injury" as used herein is a cartilage disorder or cartilage damage resulting notably from a trauma. Cartilage injuries can occur notably after traumatic mechanical destruction, notably further to an accident or surgery (for instance microfracture surgery). This term "cartilage injury" also includes chondral or osteochondral fracture and damage to meniscus. Also considered within this definition is sport-related injury or sport- related wear of tissues of the joint. The term also includes microdamage or blunt trauma, a chondral fracture, an osteochondral fracture or damage to meniscus.
- The term "osteochondral defects" (OCD) is a cartilage disorder in which defects of the cartilage cover the end of a bone in a joint. These defects are more often due to a trauma or injury, but can also be due to a pathology. OCD may lead to OA. OCD generally implies that parts of the bone is also involved, not only cartilage. If only cartilage is involved we will preferred the term "cartilage injury" (see above).
- The term "tissue engineering" encompasses also autologous chondrocyte implantation (ACI). It is also known as regenerative medicine. Cells or tissues can be cultivated either in monolayer cultures or in 3D cultures. The aim of such procedures is to repair or replace parts of or whole tissues.
- The term "graft" is related to transplantation or implantation. This procedure is also part of regenerative medicine. It includes osteochondral or cartilage (also referred to herein as osteochondral/cartilage) transplantation/implantation, such as osteochondral/cartilage autograft or osteochondral/cartilage allograft transplantation/implantation. In the frame of a graft, an explants is harvest from a mammal, either from the mammal to be treated (i.e. autograft) or from another mammal preferably of the same species (allograft). Usually, it is taken from a health cartilage section or from a healthy ostheochondral tissue.. Such graft is preferably performed at the level of the cartilage defect(s).
- The term "transplantable cartilage material" or "transplantable material" are used interchangeably. They refer to chondogenic cells, such as chondrocytes, or to osteochondral/cartilage explants that are prepared in order to be transplanted (or implanted) in a mammal in need thereof. Such transplantable material is preferably transplanted/implanted at the level of the cartilage defect(s).
- In the context of the present invention, the "efficacy" of a treatment can be measured based on changes in the thickness of the cartilage, for instance the thickness of the articular cartilage of the joint. This thickness can be assessed, for instance, through X-ray computed tomography, Magnetic Resonance Imaging (MRI) or ultrasonic measurments.
- The term "about" in "about 24, 48 or 72 hours" or in "about one day, 2 days or 3 days" encompasses changes in culture medium 24, 48 or 72 hours after supplementation in FGF-18 compound, as well as changes in culture medium 24, 48 or 72 hours +/- few hours after supplementation in FGF-18 compound (e.g. +/- 1, 2, 3 or 4 hours). Similarly, the term "about" in "about 7 days", "about one week", "about 4 weeks" or "about one month" encompasses respectively 7 days, 1 week, 4 weeks (i.e. 28 days) or one month, as well as administration separated respectively by 7 days +/- 1 or 2 days, one week +/- 1 or 2 days, 4 weeks +/- few days (e.g. +/- 1, 2, 3, 4 day(s)) or one month +/- few days (e.g. +/- 1, 2, 3, 4 day(s)). Indeed, it should be understood that, notably for a practical point of view, the changes of culture medium or the next supplementation with an FGF-18 compound cannot always be performed at exact intervals, *e.g.* renewal of the culture medium exactly 24, 48 or 72 hours after the FGF-18 compound supplementation, 4 weeks (28 days) day per day after the previous supplementation. Therefore, in the context of the invention, for instance 4 weeks means 28 days, but may also be 24, 25, 26, 27, 28, 29, 30, 31 or 32 days after the previous administration. In the context of the present invention, the term "4 weeks" is similar to the term "1 month" and they can be used interchangeably. "4 weeks" will be preferably used should one refers to "days" (e.g. 1^{st} supplementation a Monday, next supplementation a Monday 4 weeks after) and "month" will be preferably used should one refers to a "date" (e.g. 1^{st} supplementation the 1^{st} of August, next supplementation the 1^{st} of September).
- the term "cycle" means a cycle of supplementation. In the context of the present invention a weekly cycle (or a 7-days cycle) means that a culture medium will be supplemented for one day about every week (or about every 7 days) with an FGF-18 compound. Thus said cycle will include one day of culture in a supplemented medium and about 6 days of culture in a non-supplemented medium (i.e. without FGF-18). Similarly a 4-weekly cycle means that a culture medium will be supplemented for one day about every 4-weeks with an FGF-18 compound. Thus said cycle will include one day of culture in a supplemented medium and about 4 weeks of culture in a non-supplemented medium (i.e. without FGF-18). The same apply with a monthly cycle: a monthly cycle means that a culture medium will be supplemented for one day about every month with an FGF-18 compound. Thus said cycle will include one day of culture in a supplemented medium and about one month of culture in a non-supplemented medium (i.e. without FGF-18). A cycle can be repeated.

### Detailed description of the invention

Although cartilage restorative procedures such as osteochondral/cartilage grafts, and cultured cartilage implantation (e.g. ACI) are promising, integration rate or quality of the cartilage produced have to be improved. There is therefore a need of a method for improved procedure, allowing good integration and good quality of the cartilage produced (i.e. mainly hyaline cartilage). It has been surprisingly found that when FGF-18 is used in regenerative medicine (such as tissue-engineering procedures or in graft procedures), the quality of the produced cartilage is improved and there is a better integration of the cells/explants into the defects..

It is the object of the present invention to provide a process for producing a transplantable cartilage material for tissue engineering or osteochondral/cartilage graft, wherein said process comprises or consists of the steps of culturing chondrogenic cells, in 3D culture, or culturing osteochondral/cartilage explant(s), in a culture medium comprising an FGF-18 compound for a time sufficient to allow the formation of a transplantable cartilage material. Said transplantable cartilage material can be useful for treating a cartilage disorder, such as osteoarthritis, a cartilage injury (including cartilage defect) or an osteochondral defect. Preferably, the chondrogenic cells or the osteochondral/cartilage explant(s) are harvested or isolated from a mammal before expansion or culture step. Therefore, alternatively, it is an object of the present invention to provide a process for producing a transplantable cartilage material for tissue engineering or osteochondral/cartilage graft, wherein said process comprises or consists of the steps of: (a) harvesting or isolating from a mammal chondrogenic cells or osteochondral/cartilage explant(s), and (b) culturing the chondrogenic cells, in 3D culture, or culturing the osteochondral/cartilage explant(s), in a culture medium comprising an FGF-18 compound for a time sufficient to allow the formation of a transplantable cartilage material. Said transplantable cartilage material can be useful for treating a cartilage disorder, such as osteoarthritis, a cartilage injury or an osteochondral defect. Optionnaly, the FGF-18 compound can additionally be injected at the site of transplantation of the resulting cartilage material or of an osteochondral/cartilage explant, either before, at the time of or after transplantation. The scope of the invention is defined by the claims. Any references in the description to methods of treatment refer to the compounds, pharmaceutical compositions and medicaments of the present invention for use in a method for treatment of the human (or animal) body by therapy (or for diagnosis).

Herein disclosed (but not part of the invention) is also a process for regenerating cartilage in a mammal in an area of articular cartilage defect due to a cartilage disorder, said process comprising or consisting of the steps of: (a) culturing chondrogenic cells, in 3D culture, or culturing osteochondral/cartilage explant(s), in a culture medium comprising an FGF-18 compound, and (b) administering to the mammal in need thereof the cultured chondrogenic cells or osteochondral/cartilage explant(s) obtained from step (a). Said process for regenerating cartilage can be useful for treating a cartilage disorder, such as osteoarthritis, a cartilage injury or an osteochondral defect. Preferably, the chondrogenic cells or osteochondral/cartilage explant(s) are harvested or isolated from a mammal before culture step. Optionally, the FGF-18 compound can additionally be injected at the site where the culture chondrogenic cells or osteochondral/cartilage explants(s) have been administered, either before, at the time of or after admisnitration of the cells/explants. Herein disclosed (but not part of the invention) is also a process for regenerating cartilage in a mammal in an area of articular cartilage defect due to a cartilage disorder, said process comprising or consisting of the steps of: (a) culturing chondrogenic cells, in 3D culture, or culturing osteochondral/cartilage explants(s), in a culture medium, (b) administering to the mammal in need thereof the cultured chondrogenic cells or osteochondral/cartilage explants(s)obtained from step (a), and (c) injected an FGF-18 compound at the site where the cultured chondrogenic cells or the osteochondral/cartilage explant have been administered. Step (c) can be performed either before, at the time of or after administration of the cells/explants. Also disclosed is a process for regenerating cartilage in a mammal in an area of articular cartilage defect due to a cartilage disorder, said process comprising or consisting of the steps of: (a) harvesting or isolating from a mammal chondrogenic cells or osteochondral/cartilage explant(s), (b) culturing the chondrogenic cells, in 3D culture, or culturing osteochondral/cartilage explants(s)(s), in a culture medium , (c) administering to the mammal in need thereof the cultured chondrogenic cells or osteochondral/cartilage explants(s) obtained from step (b), and (d) injected an FGF-18 compound at the site where the cultured chondrogenic cells or osteochondral/cartilage explants(s) have been administered. Step (d) can be performed either before, at the time of or after administration of the cells/explants.. The scope of the invention is defined by the claims. Any references in the description to methods of treatment refer to the compounds, pharmaceutical compositions and medicaments of the present invention for use in a method for treatment of the human (or animal) body by therapy (or for diagnosis). Herein also disclosed (but not part of the invention) is a method for treating a defect in a cartilage tissue of a mammal, wherein said cartilage defect is due to a cartilage disorder, the method comprising or consisting of the following steps: (a) isolating chondrogenic cells or an osteochondral/cartilage explant from a mammal, (b) subjecting said chondrogenic cells or osteochondral/cartilage explants(s) to an in vitro or ex vivo culture, wherein said culture is performed in a cell culture medium comprising an FGF-18 compound (c) optionally repeating steps (a) and (b) to obtain a transplant material comprising the cultured chondrogenic cells or osteochondral/cartilage explants(s), and (d) transplanting the transplant material of step (c) into the defect of the mammal in need of said treatment, wherein during steps (b) and (c) the chondrogenic cells are cultured in 3-D culture. Optionnaly, the FGF-18 compound can additionally be injected at the site of transplantation, either before, at the time of or after transplantation. Herein also disclosed (but not part of the invention) is a method for treating a defect in a cartilage tissue of a mammal, wherein said cartilage defect is due to a cartilage disorder, the method comprising or consisting of the following steps: (a) isolating chondrogenic cells or an osteochondral/cartilage explant from a mammal, (b) subjecting said chondrogenic cells or osteochondral/cartilage explants(s) to an in vitro or ex vivo culture, wherein said culture is performed in a cell culture medium, (c) optionally repeating steps (a) and (b) to obtain a transplant material comprising the cultured chondrogenic cells or the osteochondral/cartilage graft, (d) transplanting the transplant material of step (c) into the defect of the mammal in need of said treatment, wherein during steps (b) and (c) the chondrogenic cells may be cultured in 3-D culture and (e) injected an FGF-18 compound at the site of transplantation. Step (e) can be performed either before, at the time of or after transplantation.

Herein also disclosed is a composition comprising a cultured mammal osteochondral/cartilage explants(s), or cultured mammal chondrogenic cells, in a medium comprising an FGF-18 compound for use in regenerative medicine, such as in tissue engineering or osteochondral/cartilage graft in a mammal in need thereof. Preferably, the mammal in need of said composition has a cartilage disorder. Preferably, the chondrogenic cells or osteochondral/cartilage explants(s) are harvested or isolated from a mammal before expansion or culture step.

It has to be understood that the transplantable cartilage material obtained according to the invention is for use in the treatment of a cartilage disorder.

In the context of the present invention as a whole, the FGF-18 compound is preferably selected from the group consisting of: a) a polypeptide comprising or consisting of the human FGF-18 mature form comprising residues 28-207 of SEQ ID NO:1, b) a polypeptide comprising or consisting of the residues 28-196 of SEQ ID NO:1, or c) a polypeptide comprising or consisting of SEQ ID NO:2. Particularly, this compound is selected from human wildtype mature FGF-18 or trFGF-18.

Herein described is an FGF-18 compound that is added in the culture medium (i.e. medium supplementation) at a concentration of 1 nanogram (ng) to 50 micrograms (µg or mcg), preferably 5 ng to 5 µg, or preferably 5 ng to 1 µg, or more preferably 10 ng to 500 µg, or even more preferably 10 ng to 100 ng per millilitre (mL) of culture medium. In a preferred embodiment the medium is supplemented with the FGF-18 compound at a concentration of about 1, 5, 10, 20, 30, 40, 50, 100, 150, 200, 250, 300, 400, 500 or 1000 ng per mL of culture medium. Preferred concentrations include 10, 20, 30, 40, 50, 100, 150 or 200 ng per mL of culture medium.

In the context of the present invention as a whole, FGF-18 is added in the medium in which the chondrogenic cells or osteochondral/cartilage explants(s) are cultured. Preferably, said FGF-18 compound is added intermittently in the culture medium, for about one day, 2 days or 3 days per week (about one week), said one-day, 2 or 3 days addition being repeated each week for at least 2 weeks of culture, at least 3 weeks of culture or at least 4 weeks of culture. Preferably, Said FGF-18 compound is added intermittently in the culture medium, for one, 2 or 3 days per week, said one-day, 2 or 3 days addition being repeated each week for 2 weeks of culture, 3 weeks of culture or 4 weeks of culture. One day is preferably to be understand as about 24 hours (*i.e.* 24 hours +/- 4 hours), two days is preferably to be understand as about 48 hours (*i.e.* 48 hours +/- 4 hours) and three days is preferably to be understand as about 72 hours (*i.e.* 72 hours +/- 4 hours). After a one-day culture with a supplemented medium, the culture is then pursued for 6 other days without the FGF-18 compound, after a 2-days culture with a supplemented medium, the culture is then pursued for 5 other days without the FGF-18 compound, and after a 3-days culture with a supplemented medium, the culture is then pursued for 4 other days without the FGF-18 compound. Said scheme corresponds to a weekly cycle. For instance, for a 1-day culture, should the FGF-18 compound being added in the culture medium a Tuesday, it is removed from said culture medium one day after said supplementation, i.e. the Wednesday. Then, the next supplementation will be done the Tuesday following the FGF-18 addition. The culture supplementation can be repeated every week (e.g. every Tuesday), according to the same scheme (i.e. one week after the previous supplementation). Should it be more convenient, the supplementation with the FGF-18 compound can be performed about one week after the previous supplementation" i.e. one week (or 7 days) +/- 1 or 2 days. For instance a supplementation can be done a Monday or a Wednesday, if the previous supplementation has been performed the previous Tuesday.

Alternatively, the FGF-18 compound can be added intermittently in the culture medium, for one, 2 or 2 days per month, said one-day, 2- or 3-days addition being repeated each month for at least 2 months of culture, at least 3 months of culture or at least 4 months of culture. For chondrogenic cell 3D culture, preferably, the FGF-18 compound is added intermittently in the culture medium, for one, two or three days per month, said one-day, 2- or 3-days addition being repeated each month for 2 months of culture, 3 months of culture or 4 months of culture. One day is preferably to be understand as about 24 hours (*i.e.* 24 hours +/- 4 hours). After the one-day, 2- or 3-days culture with a supplemented medium, the culture is then pursued for 1 month without the FGF-18 compound. Said scheme corresponds to a monthly cycle. For instance, should the FGF-18 compound being added for a one-day addition in the culture medium a of August, it is removed from said culture medium one day after said supplementation, i.e. the 2^{nd} of August. The next supplementation will be done the 1^{st} of September. The culture supplementation can be repeated every month, according to the same scheme (i.e. one month after the previous supplementation). Should it be more convenient, the supplementation with the FGF-18 compound can be performed about one month after the previous supplementation" i.e. one month +/- 1, 2, 3 or 4 days. For instance a supplementation can be done the 31 of August or the 3^{rd} of September if the previous supplementation has been performed the 1^{st} of August.

As defined above, "4 weeks" and "monthly" or "one month" are interchangeable. Therefore, according to the pending invention, the FGF-18 compound can be added intermittently in the culture or medium, for one, 2 or 3 days about every 4 weks, said one-day, 2- or 3-days addition being repeated every 4 weeks for at least 2 cycle of supplementations, at least 3 cycle of supplementations or at least 4 cycle of supplementations. Preferably, the FGF-18 compound is added intermittently in the culture or medium, for one, 2 or 3 days per month, said one-day, 2- or 3-days addition being repeated each month for 2 months of culture, 3 months of culture or 4 months of culture. One day is preferably to be understand as about 24 hours (*i.e.* 24 hours +/- 4 hours).). After the one-day, 2- or 3-days culture with a supplemented medium, the culture is then pursued for 4-weeks without the FGF-18 compound. Said scheme corresponds to a 4-weekly cycle. For instance, should the FGF-18 compound being added for a one-day addition in the culture medium a Tuesday, it is removed from said culture medium one day after said supplementation, i.e. the Wednesday. The next supplementation will be done the Tuesday 4 weeks after the 1^{st} addition. The culture supplementation can be repeated every 4-weeks, according to the same scheme (i.e. one month after the previous supplementation). Should it be more convenient, the supplementation with the FGF-18 compound can be performed about 4-weeks after the previous supplementation, i.e. 4 weeks +/- 1, 2, 3 or 4 days. For instance a supplementation can be done the Monday 28 of October or the Thursday 31 of October if the previous supplementation has been performed the Tuesday 1^{st} of October.

The processes and methods herein described will be useful for treating cartilage disorders. In particular they can be useful for treating articular cartilage defects in synovial joints that are, for instance, due to age-related superficial fibrillation, cartilage degeneration due to osteoarthritis, and chondral or osteochondral defects due to injury or disease. They may also be useful for treating joint disease caused by osteochondritis dissecans and degenerative joint diseases. In the field of reconstructive and plastic surgery, FGF-18 compounds, compositions, processes and methods according to the present invention will be useful for autogenous or allogenic cartilage expansion and transfer for reconstruction of extensive tissue defects. FGF-18 compositions can be used to repair cartilage damage in conjunction with lavage of the joint, stimulation of bone marrow, abrasion arthroplasty, subchondral drilling, or microfracture of the subchondral bone.

In a preferred embodiment, the cartilage disorder to be treated according to the invention is osteoarthritis, such as knee osteoarthritis or hip osteoarthritis. The osteoarthritis to be treated can be, for example, primary osteoarthritis or secondary osteoarthritis, as well as osteoarthritis which is classified as stage 1 to stage 4 or grade 1 to grade 6 according to the OARSI classification system. In another preferred embodiment, the cartilage disorder to be treated according to the invention is cartilage injury, including microfractures or cartilage defect, or a osteochondral defect.

In the context of the present invention as a whole, the explant is preferably a cartilage explant and the chondrogenic cells are preferably chondrocytes, chondrocytes or mesenchymal stem cells derived from mature tissues. Depending on the needs, the chondrogenic cells or the osteochondral/cartilage explants are harvest from the mammal to be treated (i.e. in need of a treatment) or from a different mammal (preferably of the same species). Said mammal is preferably a human, but alternatively can also be, without any limitation, a horse, a camel or a dog or smaller mammals such as cats, rabbits, rats or mice.

### Description of the figures:

Figure 1: Preparation of cartilage defect-repair model: (A) 8mm cartilage plug, (B) central 4mm defect creation, (C) insertion of cartilage into defect, and (D) long term culture of repair construct. OD means outer diameter and ID means inner diameter.
Figure 2: (A-C) Transverse cross sections of 3D µCT reconstruction with different treatments. (D) Integration strength of the repaired defect showing increasing strength from the control to the 1+30 treatment to the 1+6 treatment. (E) Experimental setup of the push-out testing rig. Error bars are SEM.
Figure 3: µCT scans of cartilage-to-cartilage repair constructs. Left: single µCT scan slice representative of the sample. Center: three dimensional reconstruction. Right: cross-section of the reconstruction. The µCT scans demonstrate increasing integration from control to 1+30 to 1+6 treatments
Figure 4: Treatment of the CTAs with rhFGF18
Figure 5: Cell content/CTA estimated from the DNA content/CTA after 4 weeks of treatment without rhFGF18 (CTR), in permanent presence of rhFGF18 (perm), with rhFGF18 the first week only (1w) or 1 day per week (1d/w). rhFGF18 was use at 10 or 100 ng/mL. N=4. */*** mean significantly different from the control with p<0.05 and 0.001 respectively
Figure 6: GAG and HPro/CTA content after 4 weeks of treatment without rhFGF18 (CTR), in permanent presence of rhFGF18 (perm), with rhFGF18 the first week only (1w) or 1 day per week (1d/w). rhFGF18was use at 10 or 100 ng/mL. N=4. */**/*** mean significantly different from the control with p<0.05, 0.01 and 0.001 respectively
Figure 7: Collagen type I, II and Sox9 expression as well as the Collagen II/I ratio were evaluated in CTAs after 4 weeks of culture without rhFGF18 (CTR), in permanent presence of rhFGF18 (perm), with rhFGF18 the first week only (1w) or 1 day per week (1d/w). rhFGF18 was use at 10 or 100 ng/mL.
N=4. */**/*** mean significantly different from the control with p<0.05, 0.01 and 0.001 respectively Figure 8: Bovine primary chondrocytes were cultivated one or two weeks in monolayer in absence (CTR) or in permanent presence of rhFGF18 100ng/mL. Cell concentration was determined N=6.
Collagen type I, II and Sox9 expression a N=4 was measured by quantitative Real-Time PCR.

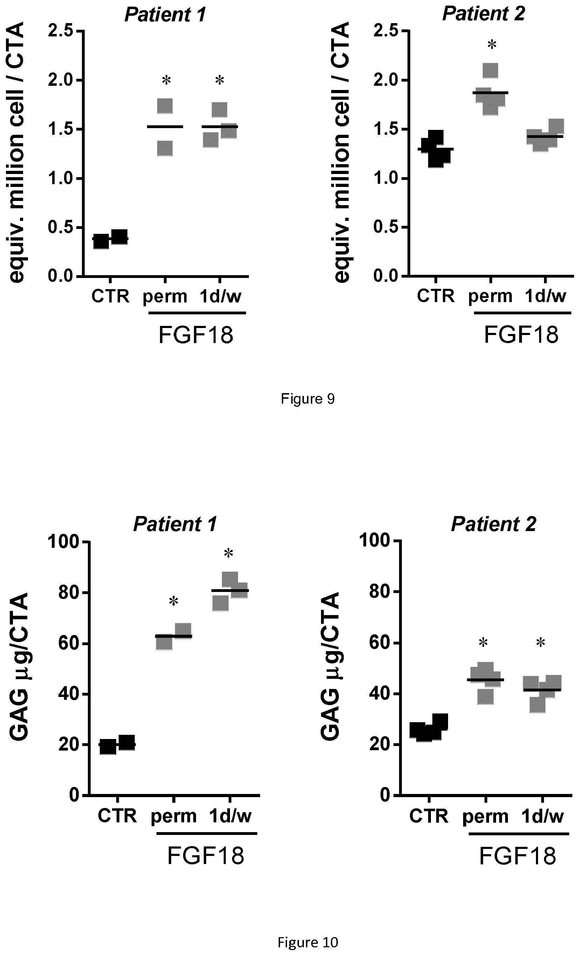

Figure 9: Cell content/CTA estimated from the DNA content/CTA after 4 weeks of treatment without rhFGF18 (CTR), in permanent presence of rhFGF18 (perm), with rhFGF18 1 day per week (1d/w). * means significantly different from the control with p<0.05.
Figure 10: GAG content after 4 weeks of treatment without rhFGF18 (CTR), in permanent presence of rhFGF18 (perm), with rhFGF18 1 day per week (1d/w). * means significantly different from the control with p<0.05.

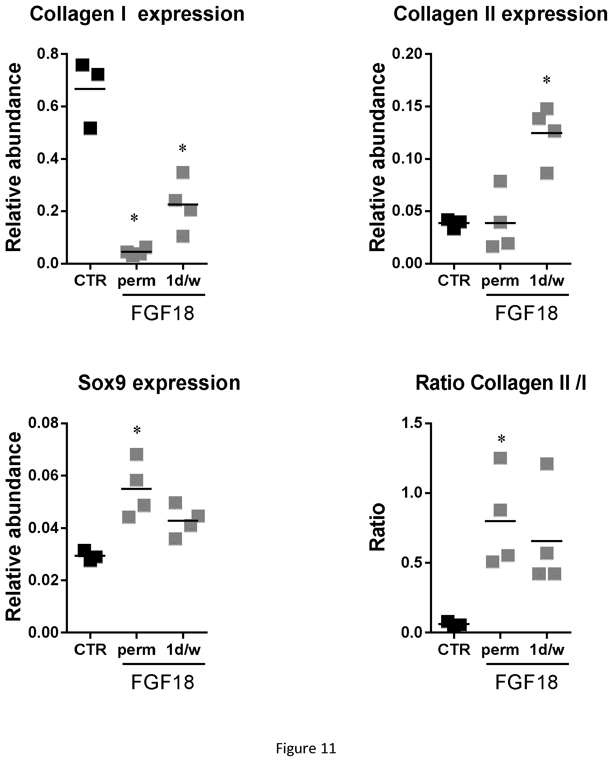

Figure 11: Collagen type I, II and Sox9 expression as well as the Collagen II/I ratio were evaluated in CTAs after 4 weeks of culture without rhFGF18 (CTR), in permanent presence of rhFGF18 (perm), with rhFGF18 1 day per week (1d/w). * means significantly different from the control with p<0.05.

### Description of the sequences:

SEQ ID NO.1: Amino acid sequence of the native human FGF-18.
SEQ ID NO.2: Amino acid sequence of the recombinant truncated FGF-18 (trFGF-18).

### Examples

### Material

The recombinant truncated FGF-18 (rhrFGF18) of the present examples has been prepared by expression in E.coli, according to the technique described in the application WO2006/063362. In the following examples rhFGF18, FGF-18 and sprifermin are used interchangeably.

### Example 1

### Methods:

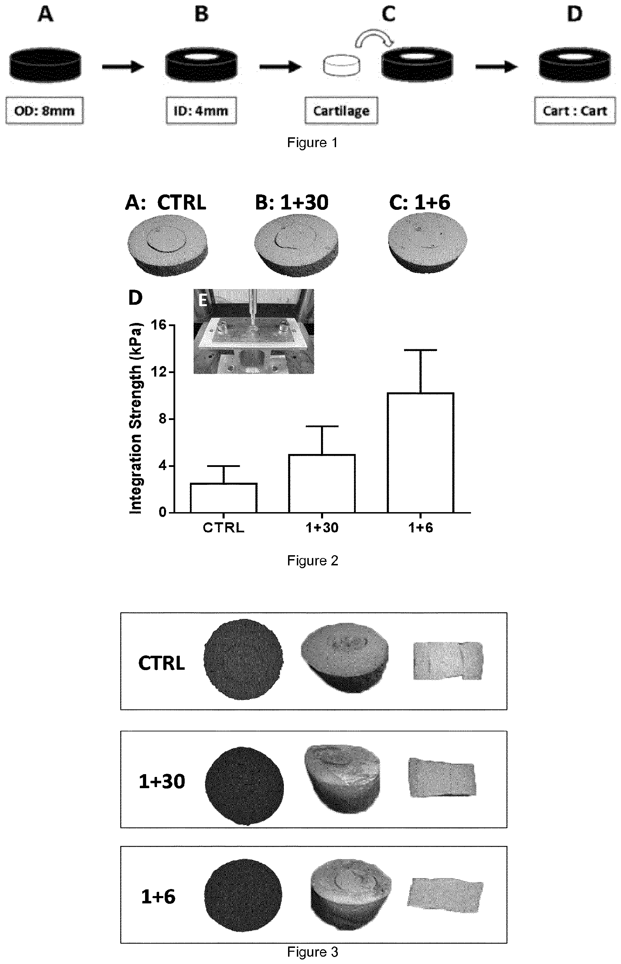

Fresh hyaline cartilage was harvested from the trochlear groove of juvenile bovine knees (3-6 months old). Cylindrical explants of 8mm (Figure 1A) were removed with a biopsy punch and cultured overnight in complete medium (DMEM 4.5g/L D-Glucose and L-Glutamine, 10% FBS, 1% PSF, 1% Fungizone, 1% MEM Vitamins, 25mM HEPES and 50µg/ml Vitamin C). Samples were trimmed of bone and defects (4mm diameter) were created to form a core and annulus repair construct (Figure 1B). Both the inner core and outer annulus were cultured separately for 24 hours before the defect was filled with the original core. Samples were then cultured in complete medium, or treated with Sprifermin (rhFGF18, 100 ng/ml). Treatments consisted of one dose of rhFGF18 for 24 hours, applied once a week (and repeated weekly) (1+6) or one 24 hour treatment followed by 1 month of culture in complete medium (1+30 days). Samples were harvested after 4 weeks of culture. Push-out mechanical testing (n=4-6) was performed (Instron 5848, Instron, Norwood, MA) using a custom testing rig (Figure 2E, [3]). Integration strength was calculated by dividing the peak force by the integration area. For 3D visualization, samples (n=6 were soaked in a modified Lugol's solution (2.5% I₂ and 5% Kl in dH₂O) for 24 hours [4] and scanned by µCT at an energy level of 55kV and intensity of 145µA with a voxel size of 6µm and 10.5µm (µCT 35 and vivaCT 40, SCANCO Medical, Wayne, PA). Scans were analyzed and reconstructed using the manufacturers software, and cross sections were used to evaluate defect integration. Additional samples (n=3) were fixed overnight in 4% PFA and analyzed histologically for cell and matrix deposition at the interface.

### Results:

The integration strength (Figure 2D) of control samples was the lowest (2.5±1.4 kPa), with progressively increasing properties with the 1+30 (monthly cycle)(5.0±2.4 kPa) and 1+6 (weekly cycle)(10.2±3.7 kPa) treatments. While the results are striking when comparing controls and treated groups, with the replicate numbers possible in this study, statistical significance was not achieved. µCT analysis of control constructs (Figure 3, top left) showed a distinct dark circle, indicating separation between the outer annulus and inner core, and thus poor integration. The 1+30 treatment (Figure 3, middle left) showed a less distinct circle, suggesting a smaller gap and greater integration, and the 1+6 treatment (Figure 3, bottom left) showed very homogenous µCT signal across the interface, indicative of the greatest degree of integration. Evidence of this increased integration was apparent on both vertical and transverse cross sections throughout the samples.

A successful cartilage repair requires that the repair material (engineered or native) be well-integrated into the surrounding cartilage to ensure continuous load transfer (and lack of stress concentrations) across the interface. In this study we investigated the potential of Sprifermin to enhance integration of cartilage in a well-defined ex vivo (explant) cartilage repair model. Sprifermin has an established pro-proliferative effect on chondrocytes (Elthworth et al., 2002), where transient (24 hour) exposure to this biologic agent elicits the most striking response. Our findings clearly demonstrate that Sprifermin improves integration strength and matrix deposition at the interface (as evidenced by contrast-enhanced µCT showing a more uniform attenuation by increase in GAG-containing proteoglycans). In this study, one 24 hour administration weekly for 4 weeks leads to an overall better outcome than one 24 hour treatment over one month This latest regimen is also be useful as, although not as good as the weekly-cycle regimen, it provides a surprising improvement compared to the control construct (i.e. in absence of sprifermin treatment). This study demonstrates for the first time that a biologic (and in particular an sprifermin) has improved the integration of cartilage surfaces in a clinically relevant repair model.

### Conclusions:

This study demonstrates that Sprifermin is able to improve the integration of cartilage surfaces in a model of cartilage repair. The findings implicate its potential usefulness in surgical procedures such as OATS and in tissue engineering approaches where cartilage like biomaterials will be required to successfully integrate with native cartilage in order to achieve clinical success.

### Example 2

### Method:

Primary osteoarthritic chondrocytes were isolated from the cartilage of patients undergoing total knee replacement. Cells were cultivated for a few days in monolayer culture first and then for one week in scaffold-free 3D culture before starting the treatment. The latter consisted in the incubation with rhFGF18 [100 ng/mL] permanently or one day/week for a total period of four weeks. Results were compared to a control culture without sprifermin. Biochemical assays, quantitative PCR (qPCR) and histology were used to characterize the 3D constructs.

### Results (data not shown):

To ensure phenotype maintenance, 3D scaffold-free culture was used to test the effect of sprifermin on hOA chondrocytes. In this setting rhFGF18 [1day/week] has been found to have a beneficial effect on the cell content and to greatly increase the size and matrix content (GAG and HPro content) of the 3D constructs. rhFGF18 was also found to decrease Collagen I expression in comparison with untreated cells..

### Conclusion:

As observed in previous studies with bovine and porcine chondrocytes, sprifermin was found to have an anabolic activity in hOA chondrocytes. The findings implicate its potential usefulness in tissue engineering approaches where cartilage like biomaterials will be required to successfully integrate with native cartilage in order to achieve clinical success

### Example 3

### Methods:

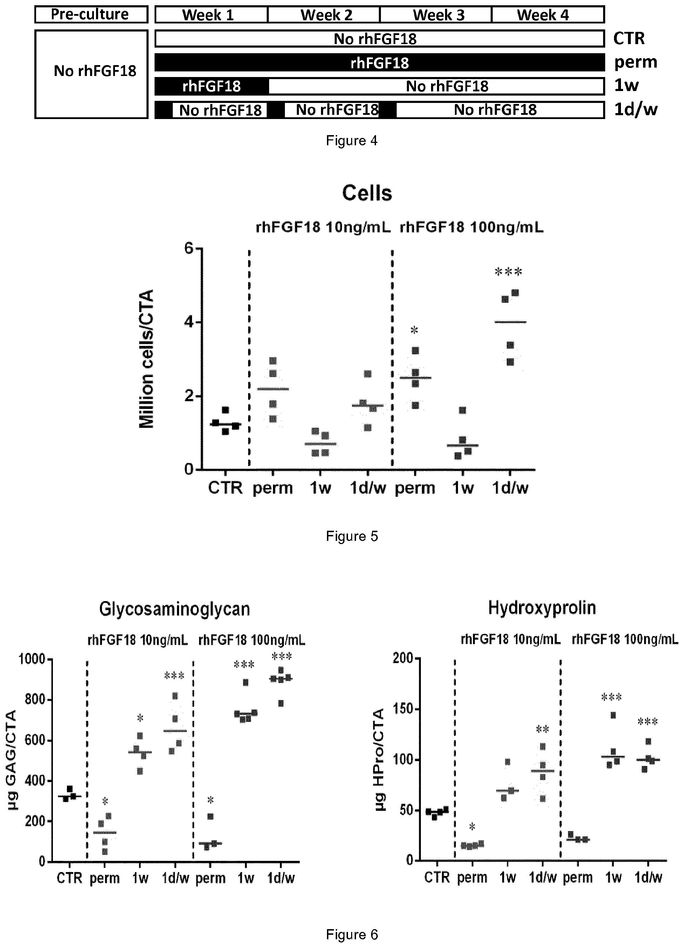

Porcine chondrocytes were isolated from the cartilage of a femoral head of a pig hip. After dissection of the joints, the cartilage was harvested and digested 45 minutes with collagenase 0.25 %. The loosened cells were discarded and the cartilage further digested overnight with collagenase 0.1 % to extract the chondrocytes. Porcine chondrocytes were cultured in suspension as CTA (Cartilage Tissue Analogs) a first week without any treatment followed by one of the following treatments: 1) four weeks of culture in permanent presence of rhFGF18 at 10 or 100 ng/mL, 2) one week of culture in presence of rhFGF18 at 10 or 100 ng/mL and subsequently three weeks without rhFGF18, 3) three weeks of culture with rhFGF18 at 10 or 100 ng/mL given 1 day per week (i.e. 24h exposure followed by 6 days without rhFGF18) and subsequently one week without rhFGF18 or 4) four weeks in absence of rhFGF18, as a control (Figure 4). At the end of the culture period, CTAs were harvested and analyzed for their GAG, hydroxyprolin and cell content. Gene expression for Collagen I, II, , and Sox9 were evaluated and histology for Safranin O, Collagen type I and II were also performed.

### Results- Effect of permanent or intermittent exposure to rhFGF18 on cell growth in CTAs

For each culture conditions, CTAs were lysed and the DNA content evaluated to calculate the number of cells/CTA (Figure 5). In the control culture (without rhFGF18) no proliferation was observed as the cell number (1.2 million) was similar to the inoculation density (1 million cell/CTA). However, as expected, the permanent presence of rhFGF18 increased chondrocyte proliferation (with 2.2 and 2.49 million cells/CTA with rhFGF18 10 and 100 ng/mL respectively). When rhFGF18 was given one week only and the chondrocytes further cultured 3 weeks without rhFGF18 (1w), no increase in the proliferation could be observed in comparison to the control. On the contrary, when rhFGF18 was given one day per week (1d/w), rhFGF18 stimulated proliferation in comparison to the control but also in comparison with the permanent exposure. The cell content /CTA reached 4 million cells/CTA with rhFGF18 100 ng/mL, one day/week, in comparison with 1.2 million in absence of rhFGF18 or 2.49 million in permanent presence of rhFGF18 100 ng/mL.

### Results- Effect of permanent or intermittent exposure to rhFGF18 on matrix production in CTAs

For each culture conditions, CTAs were digested with proteinase K and the GAG and hydroxyprolin contents were evaluated (Figure 6). GAG reflects the proteoglycan content whereas hydroxyprolin reflects the collagen content of the CTAs. As previously observed, the permanent presence of rhFGF18 decreased the GAG content/CTA (2.6 less GAG in comparison with the control) but also the hydroxyprolin content/CTA (2.1 less hydroxyprolin in comparison with the control). On the contrary when rhFGF18 is given intermittently (1/week or 1 day/week) the GAG and the hydroxyprolin content were increased. For example, when rhFGF18 100 ng/mL was given one day per week, the GAG content was increased by 2.67 and the hydroxyprolin content by 2.13 in comparison to the control.

### Results - Effect of permanent or intermittent exposure to rhFGF18 on chondrocyte phenotype in CTAs

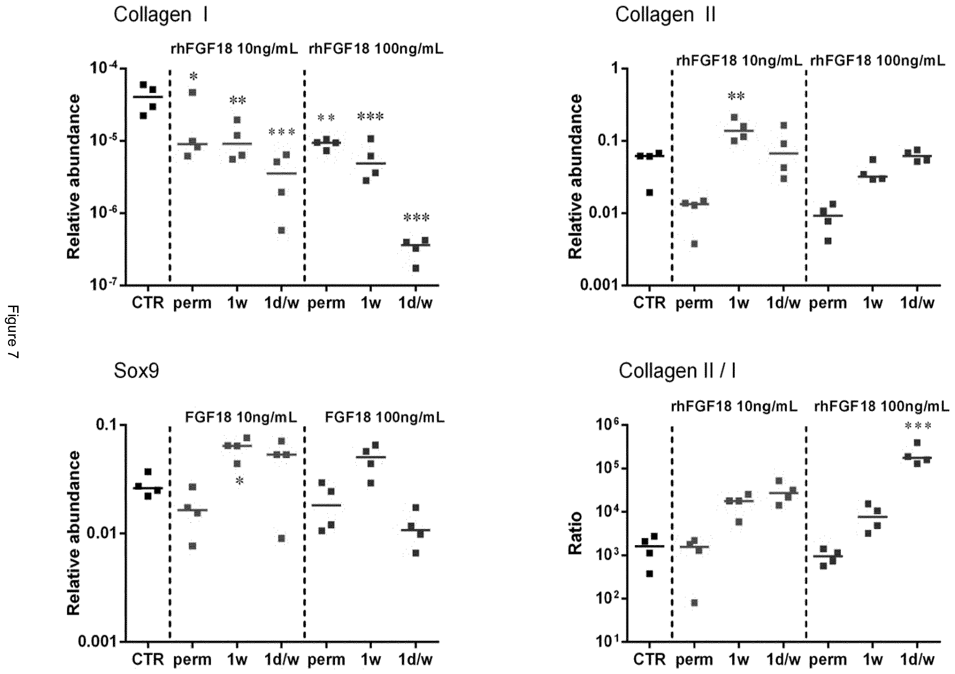

For each culture conditions, RNA was isolated from CTAs and Collagen, type I, type II, type X and Sox9 expression were analyzed by quantitative PCR (Figure 7). High Sox9 and Collagen type II expression are markers of the chondrocyte phenotype whereas Collagen type I is a marker of dedifferentiation and Collagen type X of chondrocyte hypertrophy. The ratio Collagen II / I has also been calculated. This ratio is commonly used to illustrate the phenotype maintenance (higher ratio) or phenotype loss (lower ratio) of chondrocyte in culture. In all conditions with rhFGF18, with permanent or intermittent exposure, at 10 or 100 ng/mL, Collagen type I expression was decreased. This decrease was the strongest when rhFGF18 was given one day per week at a concentration of 100 ng/mL. As an example, Collagen type I expression was decreased by 4 in comparison to the control with rhFGF18, 100 ng/mL, permanent but by 123 with rhFGF18, 100 ng/mL, given one day per week. Collagen type II was found to be decreased in permanent presence of rhFGF18 but was mostly unchanged in presence of rhFGF18 given one week (1w) or one day per week (1d/w). No important variations were observed in the Sox9 expression. The later was significantly increased (x2.2) only with rhFGF18 10 ng/mL given one week (1w). Finally, rhFGF18 permanent was found to have no effect on the Collagen II/I ratio but when rhFGF18 was given one day per week this ratio was increased by 19-fold and 138-fold in comparison to the control with rhFGF18 10 and 100 ng/mL respectively. Collagen type X was also evaluated as a marker of chondrocyte hypertrophy and was found not to be influenced by rhFGF18 in the present culture conditions.

### Results - Effect of permanent or intermittent exposure to rhFGF18 on the morphology and Collagen II and I content of CTAs (data not shown)

Histological analysis of the CTAs after 4 weeks of treatment with different rhFGF18 exposures revealed that in permanent presence of rhFGF18, CTAs were thinner and the Safranin O staining less intense in comparison with other conditions. In addition, in permanent presence of rhFGF18 a proliferative zone with a higher cell density and absence of extracellular matrix can be observed at the periphery of the constructs. On the other hand, it can also be observed that intermittent exposure to rhFGF18 resulted in thicker constructs in comparison to the control. In all conditions, Collagen type I was not detectable (not shown) while all CTAs were strongly stained for Collagen type II.

### Conclusions:

Permanent exposure to rhFGF18 stimulated chondrocyte proliferation but decreased the matrix content of the CTAs (less GAG and hydroxyprolin). Similarly both Collagen type I and II expression were decreased in comparison with the control. No significant effect of permanent exposure to rhFGF18 10 or 100 ng/mL were observed on Sox9 after 4 weeks of treatment. The histological analyses revealed that the CTAs where smaller and displayed proliferative zone devoid of ECM at the periphery of the CTAs. All these results together indicate that in permanent presence of rhFGF18 proliferation is advantaged over matrix production.

When CTAs are cultivated one week with rhFGF18, 10 or 100 ng/mL, and subsequently 3 weeks without rhFGF18, on the contrary to the permanent exposure, no stimulation of the proliferation was observed. However, the GAG and the hydroxyprolin content were found to be higher than in the control. Collagen I expression was decreased while collagen type II expression was unchanged or even slightly increased (for rhFGF18 10 ng/mL), in comparison to the control. As a consequence, the Collagen II/I ratio was increased, indicating a better phenotype maintenance. Similarly, Sox9 was also slightly increased in comparison to the control (significance for rhFGF18 10 ng/mL only). Histology revealed that CTAs were composed of a Safranin O and Collagen type II positive matrix, similarly to the control CTAs. In comparison to the control, these CTAs were also thicker, in accordance with the higher content in GAG and hydroxyprolin.

The best results regarding proliferation and matrix content were obtained when rhFGF18 100 ng/mL was given 1 day per week. For this condition Collagen type I was also the lowest and the ratio Collagen II / I the highest. However, Collagen type II and Sox 9 expression remained unchanged in comparison to the control. The CTAs were Safranin O and Collagen type II positive. As well as for the one week treatment, in comparison to the control, these CTAs were also thicker, what is also in accordance with their higher content in GAG and hydroxyprolin.

As a conclusion intermittent exposure potentiates the effects of rhFGF18 and enables to achieve increased proliferation, ECM production and promotes the chondrocyte phenotype in culture with 1day/week > 1 week >control > permanent exposure. These results support a cyclic administration of rhFGF18 for OA treatment.

### Example 4

### Methods:

Bovine chondrocytes were obtained as reported in examples 2 and 3. They were cultivated 1 or 2 weeks with rhFGF18 100 ng/mL present permanently (FGF18), or as a control in absence of FGF18 ( CTR). At the end of the culture cells were harvested and counted or lysed for RNA isolation and gene expression. Sox9, Collagen I, and II expression were evaluated by quantitative PCR .

### Results:

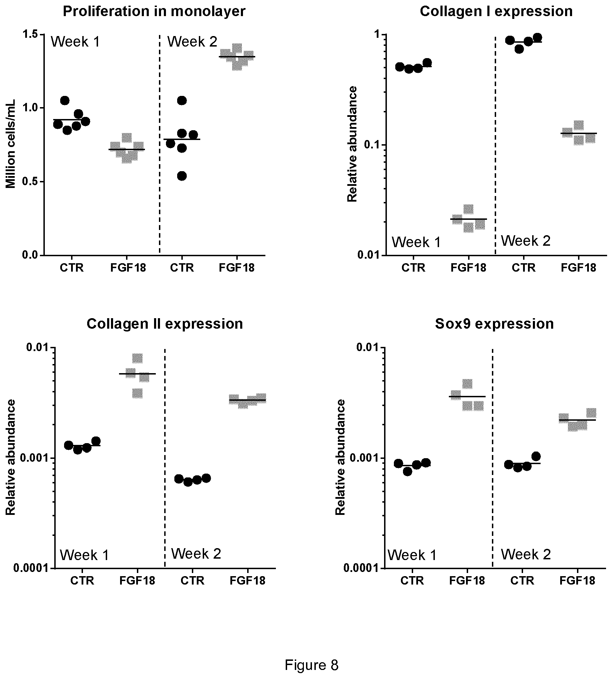

After two weeks of culture with FGF18 permanent, the cell concentration was higher than control group. Collagen type I expression was strongly repressed in presence of rhFGF18 whereas Collagen type II and Sox9 expressions were increased (Figure 8).

### Conclusion:

when chondrocyte are cultivated in monolayer, permanent exposure to rhFGF18 100 ng/mL enable to increase cell proliferation while enabling a better phenotype maintenance (Collagen II and Sox9 expression increased and Collagen I expression decreased)

### Example 5

### Methods:

The cartilage from two OA patients who underwent total knee replacement has been used. The chondrocytes were isolated as described in Example 3 and were first cultivated 3-4 days at high density in monolayer. Subsequently the chondrocytes were harvested and inoculated at 1x10⁶ cells/200µL in a 96 well plate and allowed to aggregate one week without any treatment to form CTAs. They were then further cultivated 4 weeks in absence or presence of rhFGF18 100 ng/mL according to the following treatment: 1) four weeks of culture in absence of rhFGF18 (control) 2) four weeks of culture in permanent presence of rhFGF18 (perm) and 3) four weeks of culture with rhFGF18 given 1 day per week (i.e. 24h exposure followed by 6 days without rhFGF18) (1d/w)(see Figure 4). At the end of the culture period, CTAs were harvested and analyzed for their GAG and cell content. With CTAs obtained from patient 2, gene expression for Collagen type I, type II and Sox9 were evaluated and histology for Safranin O, Collagen Type I and II were also performed.

### Results - Cell proliferation:

rhFGF18 100 ng/mL increased the proliferation of human osteoarthritic chondrocytes in 3D culture (see figure 9). For the chondrocytes coming from patient 1, in the control the number of cells/CTA was lower than the initial cell number (inoculation density was 1 million cells/CTA) indicating that many cells died. However, in presence of rhFGF18 permanent or 1 day/week 1.5 million cells/CTA could be found suggesting that these cells did not die but proliferated. For the chondrocytes coming from patient 2 a slightly increased cell number (from 1 to 1.3 million/CTA) can be observed in untreated CTA. This was further increased in presence of rhFGF18 permanent (from 1 to 1.9 million cells/CTA)

### Results - Matrix Production:

rhFGF18 100 ng/mL increased the GAG production by human osteoarthritic chondrocytes in 3D culture (see figure 10). In patient 1 wit rhFGF18 permanent and 1 day/week and in patient 2 with FGF18 permanent significantly more GAG were present in the CTAs.

### Results - Gene expression:

The chondrocyte phenotype is characterized by a low or absence of Collagen type I expression and an increased expression of Sox9 and Collagen II. This expression pattern is altered in osteoarthritic chondrocytes (see figure 11).. Indeed in untreated CTAs Collagen type I expression was higher than Collage type II expression(relative abundance of 0.67 and 0.04 respectively). rhFGF18 was able to reduce Collagen I expression while increasing Collagen II expression. As a results the ratio Collagen II/I increased 11 to 13 fold in presence of rhFGF18. In addition, rhFGF18 increased Sox9 expression, a marker of the chondrocyte phenotype.

### Results - Histology (data not shown):

In comparison to the control, the CTAs cultivated with rhFGF18 1 day/week or permanent showed an increase Safranin O staining indicating that they contained more GAG. This in accordance with the results presented in Figure 10. Collagen I staining was decreased in rhFGF18-treated cells which also corresponds well to gene expression results Figure 11. No Collagen II staining could be seen in the control culture indicating that these human osteoarthritic (hOA) chondrocytes were not able to produce a cartilage-like matrix. However, rhFGF18 1 day/week and permanent were both able to restore the ability of hOA chondrocytes to produce Collagen II.

### Conclusion:

The results obtained with chondrocytes isolated from human osteoarthritic cartilage showed that rhFGF18 was able to promote cell growth, increase hyaline-like cartilage matrix production and favor the chondrocyte phenotype. In this experiment, rhFGF18 permanent and one day/week performed equally concerning several parameters. However, regarding matrix production, rhFGF18 one day/week did slightly better (increased GAG accumulation in Patient 1 and increased Collagen II expression in Patient 2).

### References

1. Magill et al., 2011, J. orthopaedic Surg. 19(1):93-98
2. Zhang et al., 2013, Expert Opin. Biol. Ther. 13(4):527-540
3. Tang et al., 2012, Expert Opin. Biol. Ther., 12(10):1361-1382
4. Ellsworth et al., 2002, Osteoarthritis and Cartilage, 10: 308-320
5. Shimoaka et al., 2002 , JBC 277(9):7493-7500
6. WO2008023063
7. WO2004032849
8. WO9816644
9. WO2006063362
10. Custers et al., 2007, Osteoarthritis and Cartilage, 15:1241-1248
11. Lotz, 2010, Arthritis research therapy, 12:211
12. The Merck manual, 17th edition, 1999
13. Getgood et al., 2010, P116, ICRS Meeting 2010, Barcelona.
14. ICRS publication: http://www.cartilage.org/_files/contentmanagement/ICRS_evaluation.pdf, page 13
15. Bian et al., 2010, Am. J. Sports. Med, 38(1):78-85.
16. Gennero et al., 2013, Cell Biochem Funct 31 (3) 214-227
17. Mauck et al., 2006, Osteoarthritis and Cartilage, 14(2):179-189
18. Bian et al., 2008 J.Biomech., 41(6):1153-1159

## Claims

1. A process for producing a transplantable cartilage material for tissue engineering, wherein said process comprises the steps of culturing chondrogenic cells, in 3D culture, in a culture medium comprising an FGF-18 compound for a time sufficient to allow the formation of a transplantable cartilage material, wherein the FGF-18 compound is added intermittently in the culture medium, for about one, two or three days per week, said about one-day, two-days or three-days addition being repeated each week for at least 2 weeks of culture, at least 3 weeks of culture or at least 4 weeks of culture.

2. A process for producing a transplantable cartilage material for tissue engineering, wherein said process comprises the steps of culturing chondrogenic cells, in 3D culture, in a culture medium comprising an FGF-18 compound for a time sufficient to allow the formation of a transplantable cartilage material, wherein the FGF-18 compound is added intermittently in the culture medium, for about one, two or three days per month, said about one-day, two-days or three-days addition being repeated each month for at least 2 months of culture, at least 3 months of culture or at least 4 months of culture.

3. The process according to claim 1, wherein the FGF-18 compound is added intermittently in the culture medium, for one, two or three days per week, said one-day, two-days or three-days addition being repeated each week for 2 weeks of culture, 3 weeks of culture or 4 weeks of culture.

4. The process according to claim 2, wherein the FGF-18 compound is added intermittently in the culture medium, for one, two or three days per month, said one-day, two-days or three-days addition being repeated each month for 2 months of culture, 3 months of culture or 4 months of culture.

5. The process according to any one of the preceding claims, wherein the chondrogenic cells are chondrocytes or mesenchymal stem cells derived from mature tissues.

6. The process according to any one of the preceding claims, wherein the FGF-18 compound is selected from the group consisting of:
a) a polypeptide comprising or consisting of the human FGF-18 mature form comprising residues 28-207 of SEQ ID NO:1,
b) a polypeptide comprising or consisting of the residues 28-196 of SEQ ID NO:1, or
c) a polypeptide comprising or consisting of SEQ ID NO:2.

7. The process according to any one of the preceding claims, wherein the chondrogenic cells are harvest or isolated from a mammal before expansion or culture.

8. The process according to claim 7, wherein the chondrogenic cells are harvest or isolated from the mammal to be treated or from a different mammal.

9. The process according to any one of claims 7 or 8, wherein the mammal is a human.

10. Transplantable cartilage material obtained according to the process of claim 1 or claim 2 for use in the treatment of a cartilage disorder.

11. Transplantable cartilage material or regenerated cartilage, according to claim 10, wherein the cartilage disorder is osteoarthritis, cartilage injury or osteochondral defect.

## Patentansprüche

1. Verfahren zum Herstellen eines transplantierbaren Knorpelmaterials für die Gewebezüchtung, wobei das besagte Verfahren die Schritte des Kultivierens von chondrogenen Zellen in 3D-Kultur in einem Kulturmedium, welches eine FGF-18-Verbindung umfasst, eine ausreichende Zeit lang, um die Bildung eines transplantierbaren Knorpelmaterials zu ermöglichen, umfasst, wobei die FGF-18-Verbindung etwa einen, zwei oder drei Tage pro Woche lang intermittierend in das Kulturmedium zugegeben wird, wobei die besagte Zugabe über ungefähr einen Tag, zwei Tage oder drei Tage wöchentlich mindestens zwei Wochen der Kultur lang, mindestens drei Wochen der Kultur lang oder mindestens vier Wochen der Kultur lang wiederholt wird.

2. Verfahren zum Herstellen eines transplantierbaren Knorpelmaterials für die Gewebezüchtung, wobei das besagte Verfahren die Schritte des Kultivierens von chondrogenen Zellen in 3D-Kultur in einem Kulturmedium, welches eine FGF-18-Verbindung umfasst, eine ausreichende Zeit lang, um die Bildung eines transplantierbaren Knorpelmaterials zu ermöglichen, umfasst, wobei die FGF-18-Verbindung etwa einen, zwei oder drei Tage pro Monat lang intermittierend in das Kulturmedium zugegeben wird, wobei die besagte Zugabe über ungefähr einen Tag, zwei Tage oder drei Tage monatlich mindestens zwei Monate der Kultur lang, mindestens drei Monate der Kultur lang oder mindestens vier Monate der Kultur lang wiederholt wird.

3. Verfahren gemäß Anspruch 1, wobei die FGF-18-Verbindung einen, zwei oder drei Tage pro Woche lang intermittierend in das Kulturmedium zugegeben wird, wobei die besagte Zugabe über einen Tag, zwei Tage oder drei Tage wöchentlich zwei Wochen der Kultur lang, drei Wochen der Kultur lang oder vier Wochen der Kultur lang wiederholt wird.

4. Verfahren gemäß Anspruch 2, wobei die FGF-18-Verbindung einen, zwei oder drei Tage pro Monat lang intermittierend in das Kulturmedium zugegeben wird, wobei die besagte Zugabe über einen Tag, zwei Tage oder drei Tage monatlich zwei Monate der Kultur lang, 3 Monate der Kultur lang oder vier Monate der Kultur lang wiederholt wird.

5. Verfahren gemäß einem der vorangehenden Ansprüche, wobei die chondrogenen Zellen Chondrozyten oder mesenchymale Stammzellen, die aus reifen Geweben stammen, sind.

6. Verfahren gemäß einem der vorangehenden Ansprüche, wobei die FGF-18-Verbindung ausgewählt ist aus der Gruppe bestehend aus:
a) einem Polypeptid, welches die reife Form von humanem FGF-18, umfassend die Reste 28-207 von SEQ ID NO:1, umfasst oder daraus besteht,
b) einem Polypeptid, welches die Reste 28-196 von SEQ ID NO:1 umfasst oder daraus besteht oder
c) einem Polypeptid, welches SEQ ID NO:2 umfasst oder daraus besteht.

7. Verfahren gemäß einem der vorangehenden Ansprüche, wobei die chondrogenen Zellen vor der Expansion oder Kultur aus einem Säuger gewonnen oder isoliert werden.

8. Verfahren gemäß Anspruch 7, wobei die chondrogenen Zellen aus dem Säuger, der behandelt werden soll, oder aus einem anderen Säuger gewonnen oder isoliert werden.

9. Verfahren gemäß einem der Ansprüche 7 oder 8, wobei es sich bei dem Säuger um einen Menschen handelt.

10. Transplantationsfähiges Knorpelmaterial, erhalten gemäß dem Verfahren nach Anspruch 1 oder Anspruch 2 zur Verwendung bei der Behandlung eines Knorpelleidens.

11. Transplantationsfähiges Knorpelmaterial oder regenerierter Knorpel gemäß Anspruch 10, wobei es sich bei dem Knorpelleiden um Osteoarthritis, Knorpelverletzung oder osteochondralen Defekt handelt.

## Revendications

1. Procédé de production d'un matériau cartilagineux transplantable pour génie tissulaire, lequel procédé comporte les étapes consistant à cultiver des cellules chondrogènes en culture 3D, dans un milieu de culture comprenant un composé FGF-18, pendant suffisamment longtemps pour permettre la formation d'un matériau cartilagineux transplantable, et dans lequel procédé le composé FGF-18 est ajouté dans le milieu de culture de manière intermittente, durant à peu près un jour, deux jours ou trois jours par semaine, laquelle addition durant à peu près un jour, deux jours ou trois jours est répétée chaque semaine pendant au moins deux semaines de culture, au moins trois semaines de culture ou au moins quatre semaines de culture.

2. Procédé de production d'un matériau cartilagineux transplantable pour génie tissulaire, lequel procédé comporte les étapes consistant à cultiver des cellules chondrogènes en culture 3D, dans un milieu de culture comprenant un composé FGF-18, pendant suffisamment longtemps pour permettre la formation d'un matériau cartilagineux transplantable, et dans lequel procédé le composé FGF-18 est ajouté dans le milieu de culture de manière intermittente, durant à peu près un jour, deux jours ou trois jours par mois, laquelle addition durant à peu près un jour, deux jours ou trois jours est répétée chaque mois pendant au moins deux mois de culture, au moins trois mois de culture ou au moins quatre mois de culture

3. Procédé conforme à la revendication 1, dans lequel le composé FGF-18 est ajouté dans le milieu de culture de manière intermittente, durant un jour, deux jours ou trois jours par semaine, laquelle addition durant un jour, deux jours ou trois jours est répétée chaque semaine pendant deux semaines de culture, trois semaines de culture ou quatre semaines de culture.

4. Procédé conforme à la revendication 2, dans lequel le composé FGF-18 est ajouté dans le milieu de culture de manière intermittente, durant un jour, deux jours ou trois jours par mois, laquelle addition durant un jour, deux jours ou trois jours est répétée chaque mois pendant deux mois de culture, trois mois de culture ou quatre mois de culture.

5. Procédé conforme à l'une des revendications précédentes, dans lequel les cellules chondrogènes sont des chondrocytes ou des cellules souches mésenchymateuses dérivées de tissus matures.

6. Procédé conforme à l'une des revendications précédentes, dans lequel le composé FGF-18 est choisi dans l'ensemble constitué par les suivants :
a) un polypeptide comprenant la forme mature de FGF-18 humain ou consistant en cette forme, comprenant les résidus 28 à 207 de la Séquence N° 1,
b) un polypeptide comprenant les résidus 28 à 196 de la Séquence N° 1, ou consistant en ces résidus,
c) ou un polypeptide comprenant la Séquence N° 2, ou consistant en cette séquence.

7. Procédé conforme à l'une des revendications précédentes, pour lequel les cellules chondrogènes sont récoltées ou isolées à partir d'un mammifère, avant expansion ou culture.

8. Procédé conforme à la revendication 7, pour lequel les cellules chondrogènes sont récoltées ou isolées à partir du mammifère à traiter ou à partir d'un mammifère différent.

9. Procédé conforme à la revendication 7 ou 8, dans lequel le mammifère est un humain.

10. Matériau cartilagineux transplantable obtenu par un procédé conforme à la revendication 1 ou 2, pour utilisation dans le traitement d'un trouble du cartilage.

11. Matériau cartilagineux transplantable ou cartilage régénéré, conforme à la revendication 10, pour lequel le trouble du cartilage est une arthrose, une lésion du cartilage ou un défaut ostéochondral.
